# EUROPÄISCHE PATENTSCHRIFT

(11) **EP 4 271 275 B1**
(45) Veröffentlichungstag und Bekanntmachung des Hinweises auf die Patenterteilung: **17.06.2026**
(21) Anmeldenummer: 21843997.4
(22) Anmeldetag: 22.12.2021
(51) Int. Cl.: A61B 5/16

(54) **COMPUTERIMPLEMENTIERTES VERFAHREN UND VORRICHTUNG ZUR BESTIMMUNG VON REAKTIONSZEITVERLÄUFEN**
COMPUTER-IMPLEMENTED METHOD AND APPARATUS FOR DETERMINING REACTION TIME PROCESSES
PROCÉDÉ MIS EN OEUVRE PAR ORDINATEUR ET DISPOSITIF POUR DÉTERMINER DES PROFILS DE TEMPS DE RÉACTION

(30) Priorität: 29.12.2020 DE 102020135038
(43) Veröffentlichungstag der Anmeldung: 08.11.2023
(73) Patentinhaber: saniva diagnostics GmbH, 99084 Erfurt (DE)
(72) Erfinder: NISSER, Jenny, 99084 Erfurt (DE)
(74) Vertreter: Kailuweit & Uhlemann Patentanwälte Partnerschaft mbB
(86) Internationale Anmeldenummer: PCT/EP2021/087330
(87) Internationale Veröffentlichungsnummer: WO 2022/144277

(56) Entgegenhaltungen:
- JP-A- 2011 110 215
- US-A1- 2017 169 379
- BANDOW NICOLE ET AL: "Development and evaluation of a virtual test environment for performing reaction tasks", INTERNATIONAL JOURNAL OF COMPUTER SCIENCE IN SPORT, vol. 11, no. 2, 1 January 2012 (2012-01-01), pages 4 - 15, XP055910803, ISSN: 1684-4769
- GALVÁN-RUIZ JESÚS ET AL: "Perspective and Evolution of Gesture Recognition for Sign Language: A Review", SENSORS, vol. 20, no. 12, 24 June 2020 (2020-06-24), pages 3571, XP055869946, DOI: 10.3390/s20123571
- OHASHI ISSEI ET AL: "Comparison of Electromyogram During Ball Catching Task in Haptic VR and Real Environment", 7 June 2018, ADVANCES IN BIOMETRICS : INTERNATIONAL CONFERENCE, ICB 2007, SEOUL, KOREA, AUGUST 27 - 29, 2007 ; PROCEEDINGS; [LECTURE NOTES IN COMPUTER SCIENCE; LECT.NOTES COMPUTER], SPRINGER, BERLIN, HEIDELBERG, PAGE(S) 415 - 425, ISBN: 978-3-540-74549-5, XP047478327

## Beschreibung

Die Erfindung betrifft ein computerimplementiertes Verfahren zur Erfassung von motorischen Reaktionsaufgaben, wie das einhändige Fangen eines plötzlich fallenden Messkörpers.

Die motorische Funktion lässt sich durch eine Reihe von motorischen Tests quantifizieren.

Motorische Aufgaben mit erhöhter Komplexität im Sinne einer Verknüpfung verschiedener Fertigkeiten und Anforderungen zeigen hingegen frühzeitig motorische Einschränkungen an. Dazu zählt das einhändige Fangen eines plötzlich fallenden Stabes, wie es beim so genannten Fallstabtest geprüft wird. Geprüft wird hierbei das Reaktionsvermögen auf ein isoliertes subtiles optisches Signal und der Anforderung der schnellen Reizverarbeitung, wobei bei der alltagsnahen Bewegung vorrangig die Auge-Hand-Koordination und die spezifische Aufmerksamkeitslenkung die limitierenden Faktoren darstellen.

Gegenstand von US 2020 0129106A1 ist ein auf virtueller oder erweiterter Realität basierendes System zur Bewertung und Messung der Reaktionszeit. In verschiedenen Ausführungsformen beziehen sich das System, die Methoden und die Computerprogrammprodukte auf die Beurteilung und Messung der Reaktionszeit eines Patienten bzw. eines Benutzers in einer Virtual-Reality (VR)- oder Augmented-Reality (AR)-Umgebung. Das VR/AR-System kann dem Patienten/Benutzer in der VR/AR-Umgebung einen sensorischen Stimulus bieten, eine Vielzahl von Bewegungsparametern bestimmen und eine Reaktionszeit auf der Grundlage der Vielzahl von Bewegungsparametern durch Anwendung eines Zeitfenster-Auswahlmodells bestimmen.

EP 3 621 084 A1 beschreibt ein Verfahren zum Erzeugen einer Virtual-Reality-Umgebung für Übungen für einen Benutzer über eine tragbare Anzeige. Das Verfahren ist durch die folgenden Schritte gekennzeichnet:
- Auswählen einer Betriebsart der Übung;
- Anzeigen einer Virtual-Reality-Umgebung entsprechend der ausgewählten Betriebsart auf der tragbaren Anzeige, während der Benutzer die erforderlichen Schritte der Übung in der Virtual-Reality-Umgebung entsprechend der ausgewählten Betriebsart ausführt,
- Aufzeichnen parametrisierter Ergebnisse der Übung, wobei die Parameter werden gemessen als mindestens eines der folgenden Kriterien: Zeit, Entfernung, Geschwindigkeit, Genauigkeit;
- Einholen des Benutzerfeedbacks bezüglich der Übungseinheit;
- Analyse der aufgezeichneten Ergebnisse der Übung und
- auf der Grundlage des Analyseergebnisses und des Feedbacks des Benutzers Bereitstellung eines Vorschlags für eine nächste Übung.

EP 2 997 887 A1 offenbart eine virtuelle 3D-Umgebung der Hauptteile eines traditionellen CAVE-Raums. Die virtuelle 3D-Umgebung verfügt über Projektionsmittel zur Projektion von Bildern des zu prüfenden Objekts auf die Oberfläche mindestens eines Anzeigeelements der virtuellen 3D-Umgebung. Die virtuelle 3D-Umgebung umfasst ferner mindestens einen Computer zur Erzeugung von Bildern in der virtuellen Umgebung und die Aufzeichnung der Hirnfunktion mittels Elektroenzephalografie (EEG). Von Interesse sind die EEG Signale, welche während des betrachten des Objekts des Betrachters erzeugt werden. Eine vom betrachteten Objekt erzeugte Hirnreaktion wird aus den aufgezeichneten EEG-Daten mit neuroanalytischen Methoden bearbeitet, wobei der emotionale und/oder kognitive Zustand, den der Betrachter zum Zeitpunkt des Tests erlebt hat aus der Gehirnreaktion erkennbar ist. Mit diesem System ist eine direkte Korrelation aus Reiz und neurologischer Reaktion realisierbar.

Weiterhin beschreibt die US 2017/169379 A1 einen virtuellen Falltest, bei dem der Anwender ein virtuell dargestelltes Objekt fangen soll. Aus der Beschreibung geht hervor, dass der Anwender kein physisches Objekt sondern ein rein virtuelles Objekt fängt und die Bewegung optisch erfasst werden und nachträglich eine Bestimmung erfolgt, wann der Anwender das Objekt gefangen haben soll.

Die JP 2011 110215 A offenbart ebenfalls einen virtuellen Falltest, bei dem der Anwender aus einer sitzenden Position nach einem Startsignal auf einem Bildschirm ein Balkenbild angezeigt wird, welches der Anwender dann mit der dominanten Hand berühren muss. Die Zeit zwischen Startsignal und dem Berühren des Bildschirms wird erfasst und zur Bewertung von Rehaverläufen genutzt.

Das Dokument BANDOW NICOLE, *et al.* (2012) beschreibt die Erstellung einer virtuellen 3-dimensionalen Umgebung und die Testung der Reaktion von Probanden auf den Grad des Realismus der Umgebung. Dazu wurde den Probanden ein virtueller Fußball "zugeworfen" und die Zeit bis zur ersten physischen Reaktion gemessen.

Weiterhin offenbart das Dokument GALVÁN-RUIZ JESÚS, *et al.* (2020) verschiedene Möglichkeiten der Erkennung von Gesten. Neben neueren optischen Erfassungsmöglichkeiten, werden auch Datenhandschuhe als Erkennungsmöglichkeiten diskutiert.

Schließlich offenbart das Dokument OHASHI ISSEI, *et al.* (2018) ebenfalls eine virtuelle 3-dimensionale Umgebung, bei der ein Anwender ein virtuelles fallendes Objekt präsentiert wird.

Dabei wird über Kraftrückkopplung dem Anwender der Ein-druck eines Kontakts mit dem Objekt vermittelt. Zudem werden über EMG Sensoren Informationen zur Muskelspannung im Arm aufgenommen. Das System ist jedoch nicht dazu ausgelegt, eine Erfassung eines ReaktionsZeit-Verlaufs zu ermöglichen.

Jedoch besteht das Problem, dass die Lösungen des Standes der Technik spielerischen Charakter besitzen und nicht die Alltagsfunktionen bzw. sensomotorischen Fähigkeiten im Sinne einer Erfassung motorischer Parameter abprüfen. Die zugrundeliegenden motorischen Grundfähigkeiten sind somit nicht standardisiert zu messen und eine frühzeitige Erkennung von Auffälligkeiten in den motorischen Fähigkeiten und der Reizverarbeitung ist nicht möglich.

Aufgabe der Erfindung ist es, die offensichtlichen Nachteile des Standes der Technik zu überwinden und die virtuelle Anwendung eines motorischen Tests, der der Erfassung der senso-motorischen Leistungsfähigkeit bei einer Reaktionsaufgabe dient zu ermöglichen.

Gelöst wird die Aufgabe durch die Merkmale der unabhängigen Ansprüche. Bevorzugte Ausführungen sind Gegenstand der jeweils rückbezogenen Unteransprüche.

Bei einer komplexen neurokognitiven Aufgabe, wie beispielsweise dem einhändigen Fangen des plötzlich fallenden virtuellen Gegenstandes, werden motorische und kinematische Messdaten, insbesondere als zeitliche Verläufe, erfasst und ausgewertet. Der virtuelle motorische Test, bspw. als virtueller Fallstabtest, wird dabei rein softwarebasiert teilweise mit manuelle bedienbarer unterstützender Hardware-Sensorik umgesetzt. So kann die Bewegung insbesondere der Hand beim einhändigen Fangen erfasst werden. Es erscheint bspw. ein plötzlich fallender Gegenstand in einer Virtual Realtity (VR) Umgebung, welcher aus einer definierten Haltung heraus vom Benutzer gefangen werden muss. Während der Testsituation, welche das Fangen des Gegenstandes als zentrales Motiv aufweist, werden die Bewegungsver- und -abläufe von mindestens einem Sensorsystem erfasst. Mittels eines computerimplementierten Verfahrens wird die Testumgebung erzeugt und alle relevanten Daten zur Auswertung und/oder Klassifizierung ermittelt.

Dazu weist ein computerimplementiertes Verfahren zur Bestimmung von Reaktionszeitverläufen mindestens eines Anwenders mittels mindestens eines Sensorsystems bzw. einer Sensoranordnung, die folgenden Schritte auf:
i. Darstellen mindestens eines virtuellen Objektes in einem virtuellen Raum,
ii. Darstellen einer gerichteten Bewegung des virtuellen Objektes in einem Raum, wobei die Bewegung entlang einer Achse, die der x-, y- oder z-Achse eines kartesischen Koordinatensystems entspricht, bezogen auf den Anwender, verläuft,
iii. Bestimmen oder berechnen des realen zeitlichen und räumlichen Verlaufes der gerichteten Bewegung des virtuellen Objektes,
iv. Erfassen des Anwenders im realen Raum,
v. Erfassen der Bewegung oder Bewegungsabläufe des Anwenders im realen Raum,
vi. Bestimmen oder Berechnen des realen zeitlichen und räumlichen Verlaufs der gerichteten Bewegung des Anwenders
vii. Korrelieren der virtuellen und der reellen Bewegungsabläufe der Schritte iv und vi und
viii. Bestimmung der Reaktionszeitverläufe anhand der Bewegungsabläufe aus den Schritten iv und vi.

Erfindungsgemäß wirkt das computerimplementierte Verfahren zur Bestimmung von Reaktionszeitverläufen als ein Surrogat für das Interagieren mit einem Objekt in einer virtuellen Umgebung.

In Ausführungsformen wirkt das computerimplementierte Verfahren zur Bestimmung von Reaktionszeitverläufen als ein Surrogat für das einhändige Fangen eines Objekts, wie etwa eines Stabes in einer virtuellen Umgebung.

Vorteilhaft wird mit dem erfindungsgemäßen Verfahren die Reaktionszeit des Anwenders zum Interagieren mit einem sich bewegenden Objekt, wie etwa das Fangen eines fallenden Objekts, ermittelt. Dem Anwender wird dabei ein virtuelles Objekt präsentiert, welches sich entlang einer Achse, die der x-,y oder z-Achse eines kartesischen Koordinatensystems bewegt. Die Bewegung des Anwenders im realen Raum zum Interagieren mit dem virtuellen Objekt wird erfasst und mit dem virtuellen Bewegungsablauf des Objekts korreliert. Durch Bestimmung des Kollisionspunkts kann die Reaktionszeit des Anwenders bis zum Interagieren mit dem Objekt bestimmt werden.

Erfindungsgemäß dient dabei der Anwender als Bezugspunkt für das kartesische Koordinatensystem. Dadurch verläuft im Regelfall die Achse für fallende Objekte entlang der y-Achse des kartesischen Koordinatensystems in Schwerkraftrichtung. Die z-Achse verläuft demnach in Vorwärts- oder Rückwärtsrichtung des Anwenders; die x-Achse seitwärts links und rechts des Anwenders.

Die Bewegung jeweils einer Hand des Anwenders wird kurz vor, während und nach dem Interagieren des virtuellen Gegenstandes durch Sensoren einer Vorrichtung erfasst.

Dabei umfasst eine sensorische Vorrichtung zur Bestimmung von Reaktionszeitverläufen eines realen Anwenders in einer virtuellen Umgebung:
- mindestens eine Datenverarbeitungseinheit,
- mindestens ein Anzeigeelement, geeignet zur Darstellung virtueller Objekte in einem virtuellen Raum
- mindestens ein Ausgabegerät, geeignet zur Ausgabe oder Weiterleitung der ermittelten sensorischen Informationen.

In Ausführungsformen der Erfindung umfasst die sensorische Vorrichtung mindestens eine Schnittstelle zu einer Datenbank, geeignet zum Speichern und zum Vergleich der ermittelten sensorischen Daten und mindestens einem Sensorsystem.

Der Reaktionszeitverlauf wird im Folgenden jene motorische Bewegung verstanden, welche nach Empfang eines initialen Schlüsselreizes - bspw. dem Erkennen eines Signals, wie etwa eines fallenden Gegenstandes - bis hin zur vollendeten Reaktion - bspw. dem sicheren Fangen des Gegenstandes - ausgeführt wird. Der Reaktionszeitverlauf lässt durch einen Start- und einen Stopp-Zeitpunkt Rückschlüsse auf die Reaktionszeit eines Anwenders zu. Neben Start- und Stopp-Zeitpunkten werden auch Momentanwerte der Bewegung wie bspw. Beschleunigung oder Geschwindigkeit einzelner Extremitäten des Anwenders zusammengefasst, während sie die gerichtete Bewegung ausführen, welche bspw. zum Fangen des fallenden Gegenstandes notwendig ist.

Als Anwender wird im Folgenden ein Proband verstanden, welcher der eigentlichen Messung zur Bestimmung der Reaktionszeitverläufe unterzogen wird. Der Anwender liefert durch die Interaktion mit der erfindungsgemäßen Vorrichtung bzw. aus der Anwendung des erfindungsgemäßen Verfahrens Daten, welche sein Reaktionsvermögen klassifizierbar machen und ggf. auf Unregelmäßigkeiten, bspw. im Falle neurologische und/oder neurodegenerativer Erkrankungen, schließen lassen.

In Ausführungsformen der Erfindung ist das computerimplementierte Verfahren dadurch gekennzeichnet, dass aus dem zeitlichen und räumlichen Verlauf der gerichteten Bewegung des virtuellen Objektes und dem zeitlichen und räumlichen Verlauf der Bewegung mindestens eines Anwenders mindestens einen Startpunkt und mindestens einen Kollisionspunkt von mindestens einem virtuellen Objekt und dem Anwender ermittelt wird.

Dies ist vorteilhaft, da durch die Angabe eines Kollisionspunktes ein überprüfbares Kriterium geschaffen wird, um den Abschluss einer motorischen Reaktion auf einen initialen Schlüsselreiz gegeben wird. Der Kollisionspunkt ist der Zeitpunkt, an welchem eine Interaktion eines virtuellen Gegenstandes mit einem realen Anwender stattfinden würde. Wird bspw. in einer virtuellen Umgebung ein fallender Gegenstand dargestellt und ein realer Anwender beim Versuch, diesen zu fangen beobachtet werden, so wäre der Kollisionspunkt erreicht, sobald die Hände des Anwenders den fallenden Gegenstand erstmalig berühren.

In Ausführungsformen der Erfindung wird aus einem Startsignal und dem ermittelten Kollisionspunkt die Reaktionszeit des Anwenders bestimmt. Dies ist vorteilhaft, da somit die Kerninformation, die Reaktionszeit, mit einem geringeren Maß an Messunsicherheit ermittelt wird als bei einem herkömmlichen Fallstabtest bzw. bei den im Stand der Technik genannten Lösungen, da diese auf die Bestimmung eines Kollisionspunkts vollkommen verzichten.

In Ausführungsformen der Erfindung erfolgt die gerichtete Bewegung entlang einer Achse, welche als y-Achse eines kartesischen Koordinatensystems ausgebildet ist. Dabei wird das Objekt in der virtuellen Umgebung entlang der Achse bewegt. Die Bewegung des Objekts kann dabei als fallende Bewegung von oben nach unten oder als aufsteigende Bewegung von unten nach oben ausgebildet sein. Der Anwender kann dabei derart mit dem Objekt in der virtuellen Umgebung interagieren, dass dieser das Objekt während der Abwärts- oder Aufwärtsbewegung fängt. Es ist auch eine nacheinander folgende Ausführung beider Bewegungsarten denkbar. In Ausführungsformen der Erfindung kann vorgesehen sein, dass zwei Objekte in der virtuellen Umgebung dargestellt werden, wobei die Objekte eine gegenläufige Bewegung ausführen. Dabei können sich die Objekte aufeinander zu-oder voneinander wegbewegen. Dadurch lassen sich unterschiedliche Informationen nach erfolgter Interaktion bei den verschiedenen Bewegungsarten erheben, die in Kombination eine verbesserte Aussagefähigkeit zum Reaktionsvermögen des Anwenders erlauben.

In Ausführungsformen der Erfindung erfolgt die gerichtete Bewegung entlang einer Achse, welche als x-Achse eines kartesischen Koordinatensystems ausgebildet ist. Dabei kann die Bewegung des Objekts in der virtuellen Umgebung derart ausgestaltet sein, dass das Objekt von einer Seite sich zur anderen Seite bewegt. Beispielsweise kann sich das Objekt vom linken Rand des Sichtfelds des Anwenders zum rechten Rand oder von rechten Rand des Sichtfelds des Anwenders zum linken Rand bewegen. Auch ist es denkbar, dass das Objekt sich von einer mittleren Position im Sichtfeld des Anwenders zu einer linken oder rechten Seite bewegt.

In Ausführungsformen der Erfindung erfolgt eine Darstellung von zwei Objekten in der virtuellen Umgebung, wobei die beiden Objekte eine gegenläufige Bewegung ausführen. Dabei wird der Reaktionszeitverlauf des Anwenders bei der Interaktion mit beiden Objekten bestimmt und so die Komplexität der Aufgabe gesteigert. Dadurch lassen sich zusätzliche Informationen zum Reaktionsvermögen des Anwenders gewinnen.

In Ausführungsformen der Erfindung erfolgt die gerichtete Bewegung entlang einer Achse, welche als z-Achse eines kartesischen Koordinatensystems ausgebildet ist. Dabei erfolgte eine gerichtete Bewegung des Objekts im virtuellen Umfeld derart, dass das Objekt sich aus dem Hintergrund kommend auf den Anwender zubewegt. Es ist auch denkbar, dass sich das Objekt vom Anwender weg in den Hintergrund bewegt. Bei dieser Bewegung des Objekts muss der Anwender sich entscheiden, zu welchem Zeitpunkt er mit dem Objekt interagieren will, abhängig von der Reichweite des Anwenders für die Interaktion mit dem Objekt.

In Ausführungsformen erfolgt eine Darstellung von zwei Objekten in der virtuellen Umgebung, wobei die beiden Objekte eine gegenläufige Bewegung ausführen. Dabei bewegt sich ein Objekt vom Anwender weg zum Hintergrund, während ein zweites Objekt sich vom Hintergrund auf den Anwender zu bewegt. Dabei wird der Reaktionszeitverlauf des Anwenders bei der Interaktion mit beiden Objekten bestimmt und so die Komplexität der Aufgabe gesteigert. Dadurch lassen sich zusätzliche Informationen zum Reaktionsvermögen des Anwenders gewinnen.

Alternativ ist das Verfahren als variabler Reaktionstest mit neurokognitiver Aufgabe ausgestaltet. Dabei hat der Anwender die Möglichkeit sich für eine Reaktionsart mit dem Objekt in der virtuellen Umgebung zu entscheiden. Der Anwender kann dabei entscheiden, ob er mit dem Objekt interagieren oder dem Objekt aktiv ausweichen möchte. Die Aufgabe umfasst dabei die Ausführung unterschiedlicher Bewegungen auf verschiedene Signale. Die Signale können dabei variieren in der Bewegung des Objektes (y-Achse von oben nach unten und von unten nach oben, x-Achse von rechts nach links und von links nach rechts) und Art des Auftauchens des Objektes (aufpoppen, verblassen und vibrieren/ zittern). Die erforderlichen Bewegungen können umfassen: einhändige Fangbewegung mit der rechten oder linken Hand, beidhändiges Fangen, keine Bewegung, aktives Ausweichen mit geöffneter Hand, Pronation und Supination der Hand aus Neutralstellung, Fangen im Faustschluss, Fangen in Pinzetten-Griff, Fangen auf einem Finger (z.B. Fingerbeere des Zeigefingers).

Erfindungsgemäß ist mindestens ein Sensorsystem zur kontakt- bzw. berührungslosen Erfassung von Messdaten ausgebildet. Beispielsweise, aber nicht darauf beschränkt, wird die kontaktlose Erfassung durch ein Ultraschall-Echolotsystem realisiert. Dabei wird der Bereich, in dem sich ein Anwender aufhält von Ultraschallwellen durchfluten. Die Anwesenheit des Anwenders stört das Ultraschallfeld, so dass eine berührungslose Detektion ermöglicht wird.

Des Weiteren können so berührungslos die Bewegungsabläufe erfasst und somit durch die Ultraschallstreuung die Reaktionszeitverläufe aufgezeichnet werden.

Erfindungsgemäß ist das mindestens ein optisches Sensorsystem, welches zur Erfassung von Bewegungsabläufen, geeignet ist. Beispielsweise, aber nicht darauf beschränkt wird, die optische Erfassung durch ein kameraoptisches System zu erfassen. Dabei wird der Anwender durch mindestens eine Kamera, bevorzugt durch mehr als eine Kamera, zumindest teilweise erfasst, wobei die Extremitäten des Anwenders mindestens zum optisch erfassten Teil des jeweiligen Anwenders gehören. Wird nun der Moment des "Greifens" kameraoptisch erfasst, so ist in Zusammenschau mit einem synchronisierten Testverlauf zumindest die Reaktionszeit bestimmbar. Unter einem synchronisierten Testverlauf wird hierbei eine Korrelation zwischen dem Start des Testablaufs und dem gleichzeitigen Start der Überwachung des Anwenders verstanden.

Vorteilhaft wird dabei die Überwachung des Anwenders bevorzugt in Form einer digitalisierten Videoaufzeichnung durchgeführt. Somit sind die Bewegungsverläufe direkt einem Datenverarbeitungssystem zugänglich.

Erfindungsgemäß ist mindestens ein Sensorsystem geeignet, durch die Erfassung von Berührung des Anwenders Messdaten zu erheben. Dies ist Vorteilhaft, da auf diesem Wege der Kollisionspunkt durch sensorische Einrichtungen erfasst werden kann. Beispielsweise, aber nicht ausschließlich, wird der Auslöser eines Kamerasystems mit einem Berührungsempfindlichen Schalter gekoppelt. Dieser Schalter ist auf einem stabförmigen Gegenstand angeordnet, welcher in Größe und Erscheinung dem Anwender virtuell präsentierten Gegenstand entspricht. Durch Berührung des Gegenstandes während des virtuellen Testprotokolls - vorzugsweise im Moment das Fangens - wird ein kameraoptisches System gestartet bzw. die Aufnahmerate eines im Betrieb befindlichen Kamerasystems erhöht, um so eine detailreichere Aufnahme von den motorischen Abläufen des Anwenders zu bekommen. Dies ist weiterhin vorteilhaft, da so nur die wesentlichen Daten des Bewegungsablaufes erfasst werden.

In Ausführungsformen der Erfindung ist das Sensorsystem zur Erfassung der Bewegungsdaten in oder an einem zusätzlichen reellen Messgerät angeordnet. In Ausführungsformen der Erfindung umfasst das Sensorsystem weiterhin Sensoren zur Lagebestimmung im dreidimensionalen Raum. Dadurch kann mittels der Sensoren die Position des Anwenders im dreidimensionalen Raum bestimmt und in Echtzeit in der virtuellen Umgebung dargestellt werden. Dadurch kann eine reelle Darstellung der Interaktion des Anwenders mit dem virtuellen Objekt in der virtuellen Umgebung gewährleistet werden, was insbesondere bei der Reaktionszeitbestimmung von erheblicher Bedeutung ist.

In Ausführungsformen ist das Sensorsystem bspw. an der Hand mittels eines Handschuhs oder einem Arm des Anwenders durch Klettbandagen fixiert. Mittels einer Ausgabeeinheit, bspw. einer VR-Brille, wird dem Anwender die Testumgebung eines virtuellen Fallstabtests angezeigt. Das Sensorsystem umfasst hierbei beispielsweise einen Beschleunigungssensor - ohne auf diesen beschränkt zu sein, welcher durch Kopplung an eine Datenverarbeitungseinheit zur Aufzeichnung von zeitabhängigen Momentanwerten der Beschleunigung ausgebildet ist. Dienlich ist hierfür eine Art Handschuh, der selbst mit Sensoren ausgestattet ist oder einzelne Sensoren, die direkt an der Hand angebracht werden, vorzugsweise Beschleunigungssensoren und Sensoren zur Lagebestimmung im dreidimensionalen Raum. Dies ist vorteilhaft, da so die Beschleunigung einer direkten Messung zugänglich gemacht wird.

In Ausführungsformen umfasst das als Handschuh ausgebildete Sensorsystem weiterhin vibrotaktile Elemente oder Force-Feedback-Systeme, welche dem Anwender bei Fangen des virtuellen Objekts ein haptisches Feedback vermitteln. Dies ist vorteilhaft, um dem Anwender zu vermitteln, wann dieser das virtuelle Objekt gefangen hat, um ein Übersteuern des Zugreifens zu vermeiden.

In Ausführungsformen der Erfindung ist das Messgerät zur sensorischen Erfassung oder die Einhausung der sensorischen Einrichtung zur Messung der Parameter der Bewegungsabläufe in seiner Form dem virtuellen dargestellten Objekt in seiner dargestellten Form nachempfunden. Dies ist vorteilhaft, da somit für den Anwender die Illusion einer realen Maßverkörperung der Testumgebung hergestellt wird. Dies sorgt für eine unterbewusste Akzeptanz des Messaufbaus - es bewirkt die Schaffung eines sog. Perzeptes - und bietet somit weniger systematische Fehler durch unterbewusste Reaktionen wie zögern.

In Ausführungsformen ist das Messgerät vertikal ausgerichtet und stabförmig ausgebildet. Bevorzugt ist das Messgerät derart angeordnet, dass der Anwender dieses während des erfindungsgemäßen Verfahrens zur Messung des Reaktionszeitverlaufs sicher umfassen kann. Dazu wird die Positionierung des Messgeräts dem Anwender im virtuellen Raum als virtuelles Objekt dargestellt.

In Ausführungsformen der Erfindung umfasst das Messgerät weiterhin eine Positioniereinrichtung, welche eine freie Positionierung im Raum erlaubt. Entsprechende Positioniereinrichtungen sind beispielweise Roboterarm, Delta-Robot, Mehrachsensystem oder Hexapod oder Kombinationen davon.

Durch eine Kollisionspunktprognostik bzw. -bestimmung wird dafür Sorge getragen, dass durch entsprechende Positionierung des Messgeräts der Anwender genau im visuell richtigen Moment das virtuelle Objekt berührt und die richtigen fühlbaren Oberflächeneigenschaften rund um den Kollisionspunkt anzeigt, Anwender und virtuelles Objekt also miteinander synchronisiert sind. Der Anwender hat so ein Gesamtperzept, er sieht und fühlt das nun für ihn vergegenständlichte und tatsächlich existierende virtuelle Objekt, tastet aber real auf dem entsprechend positionierten Messgerät.

In Ausführungsformen ist das Messgerät derart ausgebildet, dass die Sensoren in ein flächiges Gebilde integriert sind und das flächige Gebilde nachfolgend lösbar auf einem stabförmigen Objekt anordbar ausgebildet ist.

In Ausführungsformen der Erfindung umfasst das flächige Gebilde Drucksensoren. Dadurch ist es vorteilhaft möglich neben der eigentlichen Bestimmung des Reaktionszeitverlaufs auch Informationen hinsichtlich der Griffkraft des Anwenders zu erheben. Dadurch kann die isometrische Handkraft oder aber auch die Handkraft bei einer dynamischen Bewegungsaufgabe ermittelt werden und mit altersgerechten Normwerten des Anwenders abgeglichen werden, was Hinweise auf mögliche klinische Implikationen geben kann. In Kombination mit den Ergebnissen des Reaktionszeitverlaufs können somit wichtige frühzeitige Erkenntnisse zum Status des Anwenders erhoben werden (LaStayo, P., & Hartzel, J. (1999); Chang, H., *et al.* (2015)).

In Ausführungsformen der Erfindung ist das flächige Gebilde folienförmig oder als textiles Gebilde ausgebildet. Weiterhin weist es Mittel zur lösbaren Anordnung an ein stabförmiges Objekt auf. Beispielsweise können diese Mittel zur lösbaren Anordnung, ohne darauf beschränkt zu sein, Riemchen, Bänder, Klettverschlussverbindungen oder ähnliches sein. Dadurch ist eine einfache lösbare Anordnung an einem stabförmigen Objekt möglich.

In Ausführungsformen der Erfindung umfasst das Sensorsystem zusätzlich EMG (Elektromyografie) Sensoren, welche derart ausgebildet sind, direkt die Muskelaktivität im Unterarm bei der Reaktionsleistung zu messen. Die Messdaten können drahtlos oder kabelgebunden übertragen werden. In Ausführungsformen der Erfindung können die EMG Sensoren drahtlos fungieren oder über Kabel mit dem Dummy verbunden sein.

Zur Realisierung der Erfindung ist es auch zweckmäßig, die vorbeschriebenen Ausführungsformen und Merkmale der Ansprüche zu kombinieren.

Der Erfindungsgegenstand wird im Folgenden durch nicht einschränkende Figuren und Ausführungsbeispiele näher beschrieben.

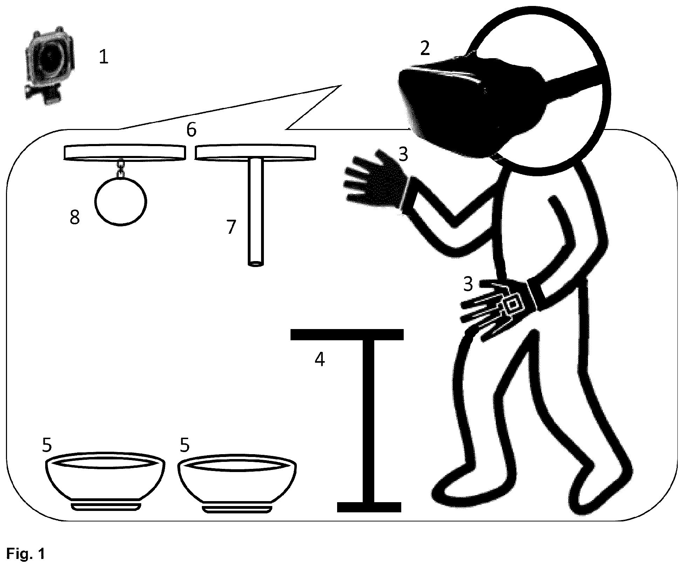

Figur 1 zeigt schematisch eine mögliche Anordnung von Sensoren und Probanden für das als "Variante 1" bezeichnete Ausführungsbeispiel des computerimplementierten Verfahrens.

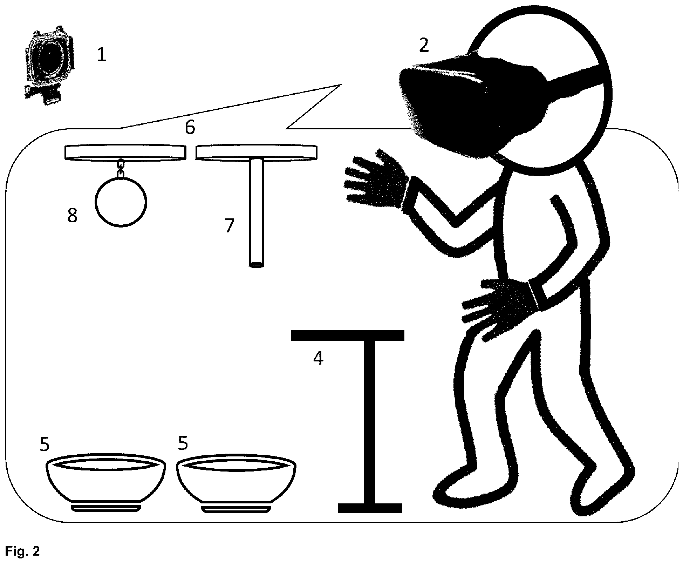

In Figur 2 wird schematisch eine mögliche Anordnung einer Testumgebung zur Ermittlung der Reaktionszeitverläufe als Ausführungsbeispiel visualisiert. Diese wird anschließend als "Variante 2" näher beschreiben.

In Figur 3 und Figur 4 ist skizzenhaft und schematisch eine weitere mögliche Anordnung zur Durchführung eines Tests zur Ermittlung der Reaktionszeitverläufe gegeben. Diese wird anschließend als "Variante 3" näher beschreiben. Dabei ist die Form des verwendeten Messkörpers in Figur 3 eine Art Joystick und in Figur 4 eine Art Slalomstange.

In Figur 5 wird schematisch eine mögliche Anordnung einer Testumgebung zur Ermittlung der Reaktionszeitverläufe als Ausführungsbeispiel dargestellt. Diese wird anschließend als "Variante 4" näher beschreiben.

Ein erstes Ausführungsbeispiel wird im Folgenden als "Variante 1" bezeichnet. Variante 1 umfasst die Anwendung eines Tests zur Ermittlung der Reaktionszeitverläufe, wobei die motorische Reaktionsfähigkeit bei einer komplexen neurokognitiven Aufgabe erfasst wird. Die für den Test nötige Testumgebung umfasst ein Sensorsystem geeignet zur Ermittlung motorischer sowie kinematischer Messdaten. In diesem Ausführungsbeispiel ist diese neurokognitive Aufgabe das einhändige Fangen eines plötzlich fallenden Gegenstandes. Der gesamte Test wird im Wesentlichen softwarebasiert umgesetzt. Dabei erscheint der plötzlich fallende Gegenstand in einer Virtual Reality (VR) Anwendung. Die Bewegung der Hand beim Fangen selbst sowie kurz davor und danach wird durch Beschleunigungssensoren an der Hand und/oder am Arm der Untersuchungsperson quantifiziert.

Der Messaufbau umfasst eine Kamera, eine VR Brille, einen Handschuh und gegebenenfalls ein Armband. Die Kamera, erfasst die Position und Bewegung der Hand im realen Raum. Das reale Bild der Hand wird in die VR Anwendung, d.h. in den virtuellen Raum, eingebunden und dem User dargestellt. Um die Bewegung der Hand während der Reaktionsaufgabe zu quantifizieren, wird der Handschuh eingesetzt. Der Handschuh ist selbst mit weiteren Sensoren ausgestattet. Im Detail handelt sich hierbei um Beschleunigungssensoren und Drucksensoren, die ihre jeweiligen Messgrößen in drei bevorzugt orthogonalen Raumrichtungen sowie in der Zeit erfassen. Ein weiteres Element zur Erfassung der Bewegung und des Beschleunigungsverhaltens der oberen Extremität im dreidimensionalen Raum ist das Armband, das die Maße einer herkömmlichen Armbanduhr hat.

Bei der Kamera handelt es sich um eine 360° Kamera, die für Actionaufnahmen geeignet ist und eine Wifi-Schnittstellt aufweist. Konkret wird das Modell: "Denver ACV-8305W 360°" verwendet. Diese Kamera lässt sich mit einem beliebigen WLAN-fähigen Endgerät steuern.

Als VR-Brille wird das Produkt: "Oculus Quest" verwendet. Oculus Quest ist eine kabellose VR-Brille, die ohne PC verwendet werden kann. Sie ist geeignet für die Anwendung bei Brillenträger und die Linsen können je nach Augenabstand angepasst werden. Letzteres ist für die Anwendung mehreren verschiedenen Personen sehr relevant.

Der Handschuh besteht aus einem gewebten Obermaterial und Sensoren (z.B. Beschleunigungsmessung und Grifffestigkeitsmessung) sind in diesen integriert. An jedem Finger und an der Handfläche sind Sensorflächen angebracht.

In das verwendete Armband sind ebenfalls Beschleunigungssensoren und eine Wifi-Kommunikationseinheit integriert. Dies ermöglicht eine drahtlose Übertragung der erhobene Parameter während der Bewegungsmessung auf die Auswerteeinheit.

Bei der Software handelt es sich um eine VR-Anwendung, die auf einem beliebigen WLANfähigem Endgerät über eine Internetseite abrufbar ist. Das Programm steuert die VR-Anwendung und das Untersuchungsprogramm wird gestartet.

Das vollständige Prozedere zur Messung der Reaktionsfähigkeit eines Probenden wird wie folgt ablaufen:
1) Optische Hilfestellung für die Einnahme der Ausgangsposition: Hierfür wird ein Objekt in Form eines Tisches eingesetzt, wodurch dem Anwender die Einnahme einer standardisierten Ausgangsposition bezogen auf die Armhaltung von 90-100° im Ellenbogengelenk einzunehmen.
2) Kontrolle der Ausgangsposition: Vor dem Testbeginn jedes einzelnen Versuchs wird die Ausgangsposition kontrolliert.
3) Simulation eines ruhenden Gegenstandes: Im oberen Teil des virtuellen Bildes wird ein ruhender Gegenstand das heißt in einer Art hängenden Position visualisiert.
4) Ausgabe eines Orientierungssignals (akustisch oder subtil visuell): Unmittelbar vor dem Beginn eines zufällig gewählten Zeitfensters wird ein Signal ausgegeben, welches ausschließlich der Orientierung des Anwenders dient soll. Das Signal kündigt den Testbeginn an, jedoch ist es ausdrücklich nicht verbunden mit dem tatsächlichen plötzlichen Fallen des Objektes. Die visuelle Aufmerksamkeit des Anwenders soll durch das Signal auf den Gegenstand gelenkt werden. Das Signal kann akustisch durch einen Piep-Ton ausgegeben werden oder subtil visuell, das heißt, nicht durch ein Lichtsignal, sondern zum Beispiel indem der visualisierte Gegenstand etwas wackelt.
5) Simulation eines vertikal fallenden Gegenstandes: Innerhalb des zufällig gewählten Zeitfensters von 6 Sekunden nach dem Orientierungssignal wird das virtuelle Bild des Gegenstandes derart simuliert, dass dieser plötzlich vertikal zu Boden fällt. Der Nutzer nimmt das plötzliche Fallen des Gegenstandes ausschließlich visuell war. Dieser Moment wird nicht durch ein zusätzliches Signal unterstützt. Der Anwender hat innerhalb des Zeitfensters die Aufgabe den simulierten Gegenstand so schnell wie möglich zu fangen. Die kinematischen Parameter werden sensorbasiert durch den Handschuh erfasst.
6) Simulation der Fangbewegung und Abbremsung der Fallbewegung des Gegenstands: Die Hand des Nutzers und dessen Bewegung wird im Bild visualisiert in Echtzeit. Die entsprechende Bewegung des Gegenstandes - insbesondere die Fangbewegung und das Abbremsen des Gegenstandes - wird simuliert. So erhält der Nutzer einen Eindruck der Fangbewegung, die der Realität möglichst stark ähnelt.
7) Auswertung der Testergebnisse: Die erhobenen Parameter, wodurch die Bewegung quantifiziert wird, werden an das gesammelt und gespeichert in einer Cloud. Schließlich erfolgt eine Auswertung der absoluten Messergebnisse unter Berücksichtigung von einer Normdatenbank von Vergleichsmessungen gesunder Personen unterschiedlichen Alters und von altersspezifischen Grenzwerten. Die Ergebnisse werden in der Output-Page ausgegeben.
8) Input-Page: Um die Testergebnisse in Relation zur Normdatenbank auswerten zu können, werden bestimmte Bezugsgrößen vom Nutzer erfasst. Diese können in einem Eingabeprotokoll (Input Page) eingegeben werden. Die Eingabe kann über zwei Wege / Modi erfolgen:
   a) Eingabe erfolgt über das Endgerät (Außensteuerung) oder
   b) Eingabe erfolgt über die VR-Anwendung (Autosteuerung).
9) Output-Page: Die gemessenen Ergebnisse von jeder Wiederholung werden erfasst und ausgegeben auf dem Endgerät und in reduzierter Form im Blickfeld des Nutzers. Zudem gibt es die Möglichkeiten der Steuerung der Anwendung/ des Tests:
   a) Der Test wird gestartet und gespeichert durch das Endgerät (Außensteuerung);
   b) Der Test kann durch den Nutzer selbst gestartet und gespeichert werden über die VR-Anwendung (Autosteuerung).
10) Speichern und Rückkoppeln: Die Untersuchungsergebnisse werden in einer Cloud und lokal auf dem Endgerät gespeichert. So kann jederzeit eine erneute Anwendung stattfinden, die entsprechend zugeordnet werden kann. Ferner ist eine Ergebnisdarstellung im Vergleich zwischen mehreren Messzeitpunkten möglich. Die spezifischen Grenzwerte und Normwerte dienen in der dargestellten Anwendung als dynamische Vergleichsgrößen. Im Falle einer therapieunterstützenden Funktion des Tests wird eine Möglichkeit der Rückkopplung eingearbeitet. Das heißt, ermittelte Daten aus der definierten Testumgebung, welche nach Abschluss des Tests durch einen Mediziner verifiziert wurden, werden einer Normdatenbank zugeführt. Die Verifizierung umfasst eine weiterführende medizinische Abklärung der neurologischen Gesundheit der Patienten in Form von weiterführender Diagnostik. Die Einstufung in "Gesund" oder "Risikopatient" wird vom Mediziner vorgenommen und im System im Nachhinein vermerkt.

In einem Weiteren, im Folgenden als "Variante 2" bezeichnetem Ausführungsbeispiel, wird analog zu Variante 1 die motorische Reaktionsfähigkeit bei einer komplexen neurokognitiven Aufgabe erfasst sowie motorische und kinematische Messdaten bei dem einhändigen Fangen eines plötzlich fallenden Gegenstandes ermittelt. Der Test wird auch in diesem Ausführungsbeispiel nahezu vollständig softwarebasiert umgesetzt. Der plötzlich fallende Gegenstand erscheint in einer Virtual Reality (VR) Anwendung, wobei die Bewegung der Hand beim Fangen selbst sowie kurz davor und danach durch Sensoren an der Hand und/oder am Arm der Untersuchungsperson quantifiziert wird.

Der Messaufbau besteht aus einer Kamera und einer VR Brille. Die Kamera, erfasst die Position und Bewegungen der Hand im realen Raum über den gesamten Messablauf hinweg. Das reale Bild der Hand wird in die VR Anwendung, d.h. in den virtuellen Raum, eingebunden und dem User dargestellt. Verschiedene Parameter werden bei der motorischen Aufgabe erfasst, die im Wesentlichen durch die Kamera erfasst werden. Dabei steht die Bewegung der Hand im dreidimensionalen Raum und das Beschleunigungsverhalten der Hand im Fokus der Betrachtung.

Bei der Kamera handelt es sich um eine 360° Kamera, die sich für Actionaufnahmen geeignet und eine Wifi-Schnittstellt aufweist. Konkret wird das Modell: Denver ACV-8305W 360-Grad-Kamera verwendet. Die Kamera lässt sich mit einem beliebigen WLAN-fähigen Endgerät steuern.

Als VR-Brille wird das Produkt: Oculus Quest verwendet. Oculus Quest ist eine kabellose VR-Brille, die ohne PC verwendet werden kann. Sie ist geeignet für die Anwendung bei Brillenträger und die Linsen können je nach Augenabstand angepasst werden. Letzteres ist für die Anwendung mehreren verschiedenen Personen sehr relevant.

Bei der Software handelt es sich um eine VR-Anwendung, die auf einem beliebigen WLANfähigem Endgerät über eine Internetseite abrufbar ist. Das Programm steuert die VR-Anwendung und das Untersuchungsprogramm wird gestartet. Dabei wird folgendes simuliert:
1) Optische Hilfestellung für die Einnahme der Ausgangsposition: Hierfür wird ein Objekt in Form eines Tisches eingesetzt, wodurch dem Anwender die Einnahme einer standardisierten Ausgangsposition bezogen auf die Armhaltung von 90-100° im Ellenbogengelenk einzunehmen.
2) Kontrolle der Ausgangsposition: Vor dem Testbeginn jedes einzelnen Versuchs wird die Ausgangsposition kontrolliert.
3) Simulation eines ruhenden Gegenstandes: Im oberen Teil des virtuellen Bildes wird ein ruhender Gegenstand das heißt in einer Art hängenden Position visualisiert.
4) Ausgabe eines Orientierungssignals (akustisch oder subtil visuell): Unmittelbar vor dem Beginn eines zufällig gewählten Zeitfensters wird ein Signal ausgegeben, welches ausschließlich der Orientierung des Anwenders dient soll. Das Signal kündigt den Testbeginn an, jedoch ist es ausdrücklich nicht verbunden mit dem tatsächlichen plötzlichen Fallen des Objektes. Die visuelle Aufmerksamkeit des Anwenders soll durch das Signal auf den Gegenstand gelenkt werden. Das Signal kann akustisch durch einen Piep-Ton ausgegeben werden oder subtil visuell, das heißt, nicht durch ein Lichtsignal, sondern zum Beispiel indem der visualisierte Gegenstand etwas wackelt.
5) Simulation eines vertikal fallenden Gegenstandes: Innerhalb des zufällig gewählten Zeitfensters von 6 Sekunden nach dem Orientierungssignal wird das virtuelle Bild des Gegenstandes derart simuliert, dass dieser plötzlich vertikal zu Boden fällt. Der Nutzer nimmt das plötzliche Fallen des Gegenstandes ausschließlich visuell war. Dieser Moment wird nicht durch ein zusätzliches Signal unterstützt. Der Anwender hat innerhalb des Zeitfensters die Aufgabe den simulierten Gegenstand so schnell wie möglich zu fangen. Die kinematischen Parameter werden sensorbasiert durch die Kamera erfasst.
6) Simulation der Fangbewegung und Abbremsung der Fallbewegung des Gegenstands: Die Hand des Nutzers und dessen Bewegung wird im Bild visualisiert in Echtzeit. Die entsprechende Bewegung des Gegenstandes - insbesondere die Fangbewegung und das Abbremsen des Gegenstandes - wird simuliert. So erhält der Nutzer einen Eindruck der Fangbewegung, die der Realität möglichst stark ähnelt.
7) Auswertung der Testergebnisse: Die erhobenen Parameter, wodurch die Bewegung quantifiziert wird, werden an das gesammelt und gespeichert in einer Cloud. Schließlich erfolgt eine Auswertung der absoluten Messergebnisse unter Berücksichtigung von einer Normdatenbank von Vergleichsmessungen gesunder Personen unterschiedlichen Alters und von altersspezifischen Grenzwerten. Die Ergebnisse in der Output-Page ausgegeben.
8) Input-Page: Um die Testergebnisse in Relation zur Normdatenbank auswerten zu können, werden bestimmte Bezugsgrößen vom Nutzer erfasst. Diese können in einem Eingabeprotokoll (Input Page) eingegeben werden. Die Eingabe kann über zwei Wege / Modi erfolgen:
   a) Eingabe erfolgt über das Endgerät (Außensteuerung) oder
   b) Eingabe erfolgt über die VR-Anwendung (Autosteuerung).
9) Output-Page: Die gemessenen Ergebnisse von jeder Wiederholung werden erfasst und ausgegeben auf dem Endgerät und in reduzierter Form im Blickfeld des Nutzers. Zudem gibt es die Möglichkeiten der Steuerung der Anwendung/ des Tests:
   a) Der Test wird gestartet und gespeichert durch das Endgerät (Außensteuerung);
   b) Der Test kann durch den Nutzer selbst gestartet und gespeichert werden über die VR-Anwendung (Autosteuerung).
10) Speichern und Rückkoppeln: Die Untersuchungsergebnisse werden in einer Cloud und lokal auf dem Endgerät gespeichert. So kann jederzeit eine erneute Anwendung stattfinden, die entsprechend zugeordnet werden kann. Ferner ist eine Ergebnisdarstellung im Vergleich zwischen mehreren Messzeitpunkten möglich. Die spezifischen Grenzwerte und Normwerte dienen in der dargestellten Anwendung als dynamische Vergleichsgrößen. Im Falle einer therapieunterstützenden Funktion des Tests wird eine Möglichkeit der Rückkopplung eingearbeitet. Das heißt, ermittelte Daten aus der definierten Testumgebung, welche nach Abschluss des Tests durch einen Mediziner verifiziert wurden, werden einer Normdatenbank zugeführt. Die Verifizierung umfasst eine weiterführende medizinische Abklärung der neurologischen Gesundheit der Patienten in Form von weiterführender Diagnostik. Die Einstufung in "Gesund" oder "Risikopatient" wird vom Mediziner vorgenommen und im System im Nachhinein vermerkt.

Ein weiteres Ausführungsbeispiel wird im Folgenden als "Variante 3" bezeichnet.

In einem weiteren Ausführungsbeispiel umfasst die Anwendung einen Test zur Ermittlung der Reaktionszeitverläufe, wobei die motorische Reaktionsfähigkeit bei einer komplexen neurokognitiven Aufgabe erfasst wird. Die für den Test nötige Testumgebung umfasst ein Sensorsystem geeignet zur Ermittlung motorischer sowie kinematischer Messdaten. In diesem Ausführungsbeispiel ist diese neurokognitive Aufgabe das einhändige Fangen eines plötzlich fallenden Gegenstandes. Der gesamte Test wird im Wesentlichen softwarebasiert umgesetzt. Dabei erscheint der plötzlich fallende Gegenstand in einer Virtual Reality (VR) Anwendung. Die Bewegung der Hand beim Fangen selbst sowie kurz davor und danach wird durch Beschleunigungssensoren an der Hand und/oder am Arm des Anwenders quantifiziert.

Der Messaufbau der Variante 3 besteht aus einer Kamera, einer VR Brille, gegebenenfalls einem höhenverstellbaren Tisch und einem Dummy als Messkörper.

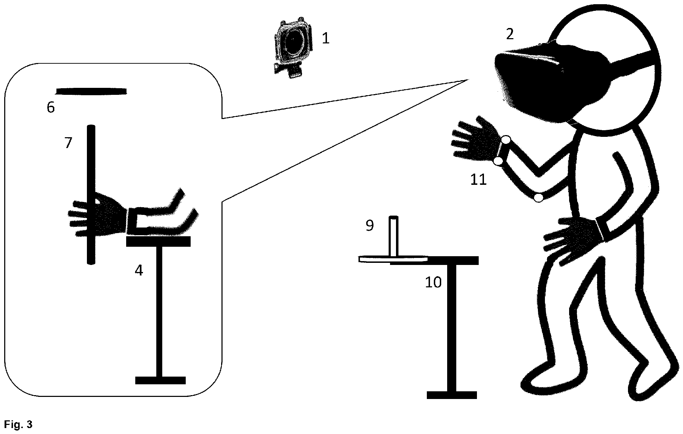

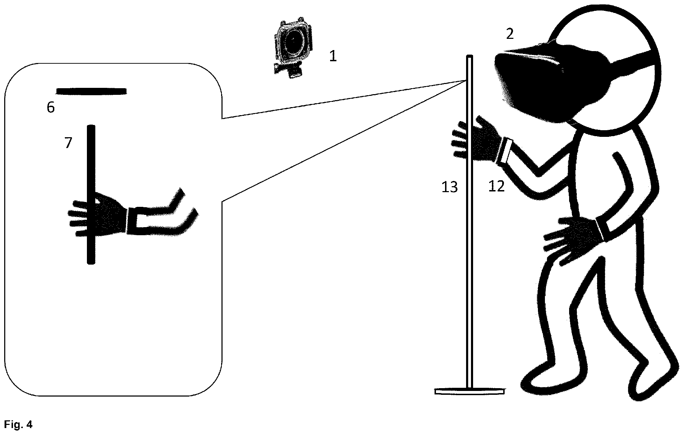

Bei dem Dummy handelt es sich beispielsweise um eine vertikal ausgerichtete Stange mit rundem Querschnitt (z.B. 3cm) und einem Standfuß, welcher auf dem höhenverstellbaren Tisch oder auf dem Boden platziert wird (Fig. 3 und 4). An der Oberfläche der Dummys ist eine Sensorfläche mit Drucksensoren angebracht, mit der die Handkraft in der dynamischen Bewegung gemessen wird.

Die Kamera erfasst die Position und Bewegung der Hand im realen Raum. Das reale Bild der Hand wird in die VR Anwendung, d.h. in den virtuellen Raum, eingebunden und dem User dargestellt.

Um die Bewegung der Hand während der Reaktionsaufgabe zu quantifizieren und das Beschleunigungsverhalten vor, während und nach der Bewegung zu erfassen, wird ein Kamera-Tracking-Verfahren eingesetzt. Dabei werden prominente anatomische Punkte der Hand und des Armes mit der Kamera erfasst und die Bewegung erfasst und aufgezeichnet. Die anatomischen Strukturen werden gegebenenfalls zuvor per Hand markiert oder per Kamera direkt erfasst. Gleiches lässt sich mit einem Armband umsetzen, das die Maße einer herkömmlichen Armbanduhr hat. Im Armband sind Beschleunigungssensoren verbaut, mit denen das Beschleunigungsverhalten vor, während und nach der Bewegung des Anwenders im dreidimensionalen Raum zeitsynchron erfasst werden kann.

Über die Kamera in der VR Brille wird die Bewegungen der Hand erfasst und das reale Bild der Hand wird in die VR Anwendung, d.h. in den virtuellen Raum, eingebunden und dem Nutzer dargestellt. Verschiedene Parameter werden bei der motorischen Aufgabe durch den Dummy erfasst. Dabei steht die Bewegung der Hand im Moment des Zufassens sowie die dynamische Handkraft im Fokus der Betrachtung.

Ebenfalls ist es denkbar, EMG Sensoren zu integrieren, die entweder drahtlos fungieren oder über Kabel mit dem Dummy verbunden sind, um direkt die Muskelaktivität im Unterarm bei der Reaktionsleistung zu messen.

Statt des Trackings der Hand und des Armes, ist auch denkbar ein Armband einzusetzen, das die Maße einer herkömmlichen Armbanduhr hat. Bei der Kamera handelt es sich um eine 360° Kamera, die für Actionaufnahmen geeignet ist und eine Wifi-Schnittstelle aufweist. Konkret wird das Modell: "Denver ACV-8305W 360°" verwendet. Diese Kamera lässt sich mit einem beliebigen WLAN-fähigen Endgerät steuern.

Als VR-Brille wird das Produkt: Oculus Quest verwendet. Oculus Quest ist eine kabellose VR-Brille, die ohne PC verwendet werden kann. Sie ist geeignet für die Anwendung bei Brillenträgern und die Linsen können je nach Augenabstand angepasst werden. Letzteres ist für die Anwendung bei / mit mehreren verschiedenen Personen sehr relevant.

In das verwendete Armband sind ebenfalls Beschleunigungssensoren und eine Wifi-Kommunikationseinheit integriert. Dies ermöglicht eine drahtlose Übertragung der erhobenen Parameter während der Bewegungsmessung an die Auswerteeinheit.

Bei den verwendeten Dummys eines Messkörpers handelt es sich in einer Version um einen vertikal ausgerichteten Stab mit rundem Querschnitt- vergleichbar mit einer Art Joystick. In einer anderen Version wird ein längerer Stab mit rundem Querschnitt und einem beschwerten kippsicheren Standfuß verwendet, - vergleichbar mit einer Art Slalomstange. Die Dummys haben folgende Konfigurationen:
Modell "Joystick", siehe Fig. 3
   - Länge: 15 cm,
   - Durchmesser im Querschnitt: 3 cm,
   - Desinfizierbare, glatte Oberfläche,
   - Ausgestattet mit Drucksensoren,
   - Wird verwendet mit einem höhenerstellbaren Tisch.
Modell "Slalomstange", siehe Fig. 4
   - Länge: 140 cm,
   - Durchmesser im Querschnitt: 3 cm,
   - Desinfizierbare, glatte Oberfläche,
   - Ausgestattet mit Drucksensoren,
   - Kippsicherer, beschwerter Standfuß, der direkt auf den Boden platziert wird.

Bei der Software handelt es sich um eine VR-Anwendung, die auf einem beliebigen WLANfähigem Endgerät über eine Internetseite abrufbar ist. Das Programm steuert die VR-Anwendung und das Untersuchungsprogramm wird gestartet. Dabei wird folgendes simuliert:
1) Optische Hilfestellung für die Einnahme der Ausgangsposition: Hierfür wird ein Objekt in Form eines Tisches eingesetzt, wodurch dem Anwender die Einnahme einer standardisierten Ausgangsposition bezogen auf die Armhaltung von 90-100° im Ellenbogengelenk ermöglicht wird. Zusätzlich wird ein höhenverstellbarer Tisch eingesetzt, an dem der Joystick angebracht ist. Der Tisch wird im virtuellen Bild für den Nutzer visualisiert zur zusätzlichen optischen Kontrolle.
2) Kontrolle der Ausgangsposition: Wird, wie in Punkt 1) beschrieben, ein realer Tisch eingesetzt, so entfällt die Kontrolle der Ausgangsposition.
3) Simulation eines ruhenden Gegenstandes: An der Stelle, an der sich auch der Messkörper/Dummy befindet, wird ein ruhender Gegenstand in einer Art hängenden Position an einer Plattform visualisiert.
4) Ausgabe eines Orientierungssignals (akustisch oder subtil visuell): Unmittelbar vor dem Beginn eines zufällig gewählten Zeitfensters wird ein Signal ausgegeben, welches ausschließlich der Orientierung des Anwenders dienen soll. Das Signal kündigt den Testbeginn an, jedoch ist es ausdrücklich nicht verbunden mit dem tatsächlichen plötzlichen Fallen des Objektes. Die visuelle Aufmerksamkeit des Anwenders soll durch das Signal auf den Gegenstand gelenkt werden. Das Signal kann akustisch durch einen Piep-Ton ausgegeben werden oder subtil visuell, das heißt, nicht durch ein Lichtsignal, sondern zum Beispiel indem der visualisierte Gegenstand sich minimal bewegt.
5) Simulation eines vertikal fallenden Gegenstandes: Innerhalb des zufällig gewählten Zeitfensters von 6 Sekunden nach dem Orientierungssignal wird das virtuelle Bild des Gegenstandes derart simuliert, dass dieser plötzlich vertikal zu Boden fällt. Der Nutzer nimmt das plötzliche Fallen des Gegenstandes ausschließlich visuell war. Dieser Moment wird nicht durch ein zusätzliches Signal unterstützt. Der Anwender hat innerhalb des Zeitfensters die Aufgabe, den simulierten Gegenstand so schnell wie möglich zu fangen ohne den Kontakt zwischen Arm und Tisch aufzulösen. Die Reaktionsleistung und die dynamische Handkraft werden durch die Sensoren, die im Dummy/Messkörper verbaut sind (z.B. Drucksensoren) erfasst und drahtlos übertragen.
6) Simulation der Fangbewegung und Abbremsung der Fallbewegung des Gegenstands: Die Hand des Nutzers und dessen Bewegung wird im Bild in Echtzeit visualisiert. Die entsprechende Bewegung des Gegenstandes - insbesondere die Fangbewegung und das Abbremsen des Gegenstandes - wird simuliert. So erhält der Nutzer einen Eindruck der Fangbewegung, die der Realität möglichst stark ähnelt.
7) Auswertung der Testergebnisse: Die erhobenen Parameter, durch welche die Bewegung quantifiziert wird, werden gesammelt und in einer Cloud gespeichert. Schließlich erfolgt eine Auswertung der absoluten Messergebnisse unter Berücksichtigung einer Normdatenbank von Vergleichsmessungen gesunder Personen unterschiedlichen Alters und von altersspezifischen Grenzwerten. Die Ergebnisse werden in der Output-Page ausgegeben.
8) Input-Page: Um die Testergebnisse in Relation zur Normdatenbank auswerten zu können, werden bestimmte Bezugsgrößen vom Nutzer erfasst. Diese können in einem Eingabeprotokoll (Input Page) eingegeben werden. Die Eingabe kann über zwei Wege / Modi erfolgen:
   a) Eingabe erfolgt über das Endgerät (Außensteuerung) oder
   b) Eingabe erfolgt über die VR-Anwendung (Autosteuerung).
9) Output-Page: Die gemessenen Ergebnisse von jeder Wiederholung werden erfasst und ausgegeben auf dem Endgerät und in reduzierter Form im Blickfeld des Nutzers. Zudem gibt es die Möglichkeiten der Steuerung der Anwendung / des Tests:
   a) Der Test wird gestartet und gespeichert durch das Endgerät (Außensteuerung);
   b) Der Test kann durch den Nutzer selbst gestartet und gespeichert werden über die VR-Anwendung (Autosteuerung).
10) Speichern und Rückkoppeln: Die Untersuchungsergebnisse werden in einer Cloud und lokal auf dem Endgerät gespeichert. So kann jederzeit eine erneute Anwendung stattfinden, die entsprechend zugeordnet werden kann. Ferner ist eine Ergebnisdarstellung im Vergleich zwischen mehreren Messzeitpunkten möglich. Die spezifischen Grenzwerte und Normwerte dienen in der dargestellten Anwendung als dynamische Vergleichsgrößen. Im Falle einer therapieunterstützenden Funktion des Tests wird eine Möglichkeit der Rückkopplung eingearbeitet. Das heißt, ermittelte Daten aus der definierten Testumgebung, welche nach Abschluss des Tests durch einen Mediziner verifiziert wurden, werden einer Normdatenbank zugeführt. Die Verifizierung umfasst eine weiterführende medizinische Abklärung der neurologischen Gesundheit der Patienten in Form von weiterführender Diagnostik. Die Einstufung in "Gesund" oder "Risikopatient" wird vom Mediziner vorgenommen und im System im Nachhinein vermerkt.

In einem weiteren Ausführungsbeispiel der Erfindung wird ein komplexer Reaktionstest in einer virtuellen Umgebung und unter Einbezug von Sensorsystemen durchgeführt. Dieses Ausführungsbeispiel wird im Folgenden als "Variante 4" bezeichnet. Der Messaufbau der Variante 4 besteht aus einer Kamera, einer VR Brille und EMG-Sensoren. Mit diesem Aufbau werden unterschiedliche motorische und kinematische Messdaten erhoben, um die Reaktionsleistung des Anwenders zu quantifizieren. Der Reaktionstest grenzt sich gegenüber den ersten beiden Ausführungsbeispielen dadurch ab, dass die auszuführende Bewegung nicht auf das einhändige Fangen eines herabfallenden Objekts beschränkt ist. Weiterhin sind komplexe Reaktionsaufgaben umzusetzen, indem der Anwender auf unterschiedliche Reize oder Signale hin mit verschiedenen motorischen Handlungen reagiert.

Als VR-Brille wird das Produkt: Oculus Quest verwendet. Oculus Quest ist eine kabellose VR-Brille, die ohne PC verwendet werden kann. Sie ist geeignet für die Anwendung bei Brillenträgern und die Linsen können je nach Augenabstand angepasst werden. Letzteres ist für die Anwendung bei mehreren verschiedenen Personen sehr relevant.

Als Oberflächen-EMG (Elektromyographie) Sensoren werden die kabelgebundenen oder kabellosen Modelle von DataLITE der Firma Biometrics Ltd. verwendet. Sie eignen sich für die Erfassung der Muskelaktivität der oberflächlichen Muskulatur, wie der des Unterarms, die bei der Bewegung der Finger angesprochen wird. Die kabellosen Sensoren ermöglichen eine Muskelaktivitätsmessung mit einer Reichweite von bis zu 30 Metern vom Empfänger. Dies ist sehr vorteilhaft für Anwendungen bei Bestimmung von Bewegungsabläufen oder Zeit-Reaktionsverläufen.

Im Folgenden wird der komplexe Reaktionstest detailliert beschrieben:
Der Anwender hat die Aufgabe auf unterschiedliche Reize hin verschiedene motorische Handlungen mit der Hand bzw. der oberen Extremität auszuführen. Dabei werden zahlreiche Parameter erfasst, wie die Reaktionszeit, die Muskelaktivität und die Bewegung sowie das Beschleunigungsverhalten der Hand und des Armes im dreidimensionalen Raum. Der motorische Test wird im Wesentlichen softwarebasiert umgesetzt. Dabei erscheinen verschiedene Gegenstände in einer Virtual Reality (VR) Anwendung.

Der komplexe Reaktionstest umfasst die Durchführung verschiedener Reaktionsaufgaben. Dabei stehen unterschiedliche Objektformen zur Auswahl, die jeweils bestimmte Bewegungen erfordern. Die Signale/ Reize variieren nach der Art des Erscheinens des Objektes und der Bewegung des Objektes. Aus der Kombination aus Objektform, Objektbewegung oder - erscheinung ergeben sich bestimmte motorische Handlungsaufgaben, die so schnell wie möglich vom Anwender auszuführen sind.

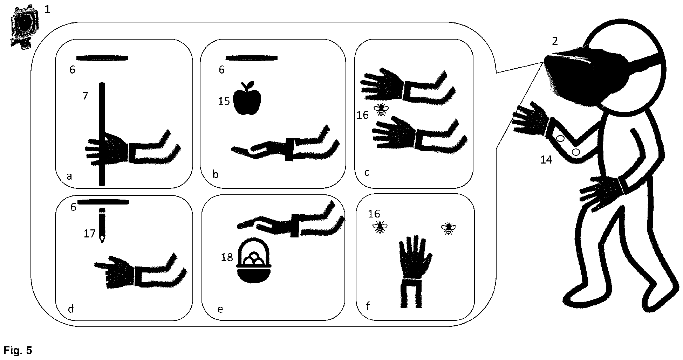

Objektformen: Das virtuelle Objekt variiert in seiner Form zwischen Apfel, Stange, Bleistift und Korb und Fliege. Endsprechend dieses Formunterschiedes werden unterschiedliche Bewegungen der Hand erforderlich:
- Der Apfel (Fig. 5b) wird mit angewinkeltem Unterarm (90-100° im Ellenbogengelenk) und der Supinationsstellung in der Hand gegriffen/ ergriffen. Der Apfel wird einhändig gefangen.
- Der Korb (Fig. 5e) wird in der Pronationsstellung in der Hand gegriffen/ ergriffen. Der Korb wird einhändig gefangen.
- Die Stange (Fig. 5a) wird in der Neutralstellung in der Hand gegriffen/ ergriffen. Die Stange wird einhändig, beidhändig simultan oder beidhändig gefangen. Zu Beginn der Bewegung sind die Hände geöffnet und ein kompletter Faustschluss wird durchgeführt.
- Der Bleistift (Fig. 5d) wird in der Neutralstellung in der Hand gegriffen/ ergriffen. Der Bleistift wird einhändig gefangen. Zu Beginn der Bewegung sind die Hände geöffnet und der Bleistift wird im Pinzettengriff zwischen Daumen und Zeigefinger gefangen.
- Die Fliege (Fig. 5c und 5f) wird zwischen den Handflächen gefangen. Zu Beginn der Bewegung sind die Hände in Neutralstellung geöffnet, die Arme vor dem Körper ausgestreckt und die Handflächen zueinander ausgerichtet. Die Fliege wird durch schnelles gegeneinanderschlagen der Handflächen gefangen.

**Objektbewegung und -erscheinung:** Die Art des Erscheinens der Objekte reicht von plötzlichem Erscheinen bis hin zu Verblassen, Vibrieren und Rotieren. Weiterhin können die Objekte auf verschiedene Weise in drei Dimensionen bewegt werden (von unten nach oben, von oben nach unten, von rechts nach links, von links nach rechts). Aus diesen Bewegungen und Erscheinungen des Objektes ergeben sich die unterschiedlichen Signale/ Reize, auf die der Anwender später mit der passenden motorischen Aktion reagieren muss.

**Reaktionsaufgaben:** Die auszuübenden Reaktionsaufgaben umfassen dabei:
- Fangen des sich plötzlich bewegenden Objektes mit einer Hand oder simultan mit beiden Händen
- Ausweichen eines sich bewegenden Objektes mit einer Hand oder beiden Händen
- Zusammenführen beider Handflächen um ein bewegliches Objekt (in die Hände klatschen)
- Berühren einer Zielfläche mit der geöffneten und gestreckten Hand einhändig, alternierend mit der rechten und linken Hand oder gleichzeitig mit beiden Händen.

Die Aufgabenstellungen der hier genannten Bewegungsformen können auch in Kombination erscheinen. Die Reaktionsaufgaben können mit und ohne Objektbewegung ausgeführt werden.

Nachfolgend wird ein beispielhafter Ablauf des komplexen Reaktionstest beschrieben.

### Aufgabe 1:

| Objektform | Apfel (wie in Fig. 5b), der an der Decke hängt und plötzlich herabfällt |
|---|---|
| Erforderliche Bewegung | Fangen in Supination mit der dominanten Hand oder Ausweichen und Hand zurückziehen |
| Objektbewegung | (A) Apfel fällt plötzlich herab ohne Zusatzsignal oder |
| | (B) vibriert vor dem herabfallen |
| Reaktionsaufgabe | bei (A) Apfel mit der dominanten Hand fangen |
| | bei (B) Apfel ausweichen |

### Aufgabe 2:

| Objektform | Bleistift (wie in Fig. 5d) oder Stange (wie in Fig. 5a) |
|---|---|
| Erforderliche Bewegung | Fangen mit Pinzettengriff mit einer Hand oder Fangen im klassischen Faustschluss mit einer Hand |
| Objektbewegung | (A) Bleistift bewegt sich von rechts in Richtung Körpermittel des Anwenders oder |
| | (B) Bleistift bewegt sich von links in Richtung Körpermittel des Anwenders oder |
| | (C) Stange bewegt sich von rechts in Richtung Körpermittel des Anwenders oder |
| | (D) Stange bewegt sich von links in Richtung Körpermitte des Anwenders oder |
| Reaktionsaufgabe | bei (A) Bleistift mit der linken Hand fangen im Pinzettengriff |
| | bei (B) Bleistift mit der rechten Hand fangen im Pinzettengriff |
| | bei (C) Stange mit der linken Hand fangen im klassischen Griff mit Faustschluss |
| | bei (D) Stange mit der rechten Hand fangen im klassischen Griff mit Faustschluss |

### Aufgabe 3:

| Objektform | Fliege (wie in Fig. 5c und Fig. 5f) |
|---|---|
| Erforderliche Bewegung | Handflächen beider Hände zusammenführen/ in die Hände klatschen Berühren einer Zielfläche mit offenen Handflächen |
| Objektbewegung | (A) Fliege bewegt sich durch den Raum und kommt auf den Anwender zu oder |
| | (B) Fliege bewegt sich nicht mehr oder |
| | (C) zwei Fliegen tauchen gleichzeitig (verblassen) im Bild auf |
| Reaktionsaufgabe | bei (A) Handflächen beider Hände zusammenführen und Fliege darin fangen |
| | bei (B) Fliege mit geöffneter Handfläche berühren |
| | bei (C) beide Fliegen gleichzeitig mit jeweils einer Hand berühren |

### Aufgabe 4:

| Objektform | Fliege (wie in Fig. 5f) |
|---|---|
| Erforderliche Bewegung | Berühren einer Zielfläche mit offenen Handflächen |
| Objektbewegung | zwei Fliegen werden dargestellt ohne Bewegung |
| Reaktionsaufgabe | Der Anwender wird mit einer Hand im Wechsel beide Fliegen so oft und so schnell wie möglich innerhalb von 30 Sekunden berühren. |

### Zitierte Nichtpatentliteratur

LaStayo, P., & Hartzel, J. (1999). Dynamic versus static grip strength: how grip strength changes when the wrist is moved, and why dynamic grip strength may be a more functional measurement. Journal of Hand Therapy, 12(3), 212-218.
Chang, H., Chen, C. H., Huang, T. S., & Tai, C. Y. (2015). Development of an integrated digital hand grip dynamometer and norm of hand grip strength. Bio-Medical Materials and Engineering, 26, S611-S617. https://doi.org/10.3233/BME-151352

### Bezugszeichen

- 1: Kamera
- 2: VR-Brille
- 3: Handschuhe und Armband ausgestattet mit Sensoren
- 4: Hilfestellung für den Anwender zum Einnehmen der Ausgangsposition als virtuelles Bild
- 5: Auffangvorrichtung als virtuelles Bild
- 6: Aufhängung der Messgegenstände als virtuelles Bild
- 7: Virtuelles Bild des Messkörper in Form eines Stabes mit rundem Querschnitt
- 8: Virtuelles Bild Messkörper in Form einer Kugel
- 9: Joystick/Messkörper in Form eines Stabes mit rundem Querschnitt als reale Verkörperung
- 10: Hilfestellung für den Anwender zum Einnehmen der Ausgangsposition als reale Verkörperung
- 11: Tracking-Punkte für Bewegungsmessung im dreidimensionalen Raum
- 12: Armband für Bewegungsmessung im dreidimensionalen Raum
- 13: Slalomstange/Messkörper in Form eines Stabes mit rundem Querschnitt als reale Verkörperung
- 14: Elektromyografie (EMG) Sensoren
- 15: Virtuelles Bild Objekt in Form eines Apfels
- 16: Virtuelles Bild Objekt in Form einer Fliege
- 17: Virtuelles Bild Objekt in Form eines Bleistiftes
- 18: Virtuelles Bild Objekt in Form eines Korbes

## Patentansprüche

1. Computerimplementiertes Verfahren zur Bestimmung von Reaktionszeitverläufen mindestens eines Anwenders mittels mindestens eines Sensorsystems, aufweisend die folgenden Schritte:
i. Darstellen mindestens eines virtuellen Objektes in einem virtuellen Raum,
ii. Darstellen einer gerichteten Bewegung des virtuellen Objektes in einem Raum, wobei die Bewegung entlang einer Achse, die der x-, y- oder z-Achse eines kartesischen Koordinatensystems entspricht, bezogen auf den Anwender, verläuft,
iii. Bestimmen oder berechnen des realen zeitlichen und räumlichen Verlaufes der gerichteten Bewegung des virtuellen Objektes,
iv. Erfassen des Anwenders im realen Raum,
v. Erfassen der Bewegung oder Bewegungsabläufe des Anwenders im realen Raum, wobei mindestens ein Sensorsystem zur berührungslosen Erfassung von Messdaten ausgebildet ist, wobei das mindestens eine Sensorsystem ein optisches Sensorsystem, geeignet zur Erfassung von Bewegungsabläufen, ist und mindestens ein Sensorsystem geeignet ist, durch die Erfassung von Berührung des Anwenders Messdaten zu erheben,
vi. Bestimmen oder Berechnen des realen zeitlichen und räumlichen Verlaufs der gerichteten Bewegung des Anwenders
vii. Korrelieren der virtuellen und der reellen Bewegungsabläufe der Schritte iv und vi und
viii. Bestimmung der Reaktionszeitverläufe anhand der Bewegungsabläufe aus den Schritten iv und vi.

2. Computerimplementiertes Verfahren nach Anspruch 1, **dadurch gekennzeichnet, dass** aus dem zeitlichen und räumlichen Verlauf der gerichteten Bewegung des virtuellen Objektes und dem zeitlichen und räumlichen Verlauf der Bewegung mindestens eines Anwenders mindestens ein Startpunkt und mindestens ein Kollisionspunkt von mindestens einem virtuellen Objekt und dem Anwender ermittelt wird.

3. Computerimplementiertes Verfahren nach Anspruch 2, **dadurch gekennzeichnet, dass** aus dem ermittelten Kollisionspunkt die Reaktionszeit des Anwenders ermittelt wird.

4. Vorrichtung zur Bestimmung von Reaktionszeitverläufen eines realen Anwenders in einer virtuellen Umgebung nach einem Verfahren nach Anspruch 1 bis 3, umfassend:
- mindestens eine Datenverarbeitungseinheit,
- mindestens ein Sensorsystem,
- mindestens ein Anzeigeelement, geeignet zur Darstellung virtueller Objekte in einem virtuellen Raum,
- mindestens ein Ausgabegerät, geeignet zur Ausgabe oder Weiterleitung der ermittelten sensorischen Informationen,
- mindestens eine Schnittstelle zu einer Datenbank, geeignet zum Speichern und zum Vergleich der ermittelten sensorischen Daten und mindestens einem Sensorsystem., wobei das Sensorsystem zur Erfassung der Bewegungsdaten in oder an einem zusätzlichen reellen Messgerät angeordnet, wobei
- - das Sensorsystem als Handschuh ausgebildet ist oder das Messgerät in seiner Form dem virtuellen Objekt in seiner dargestellten Form nachempfunden ist.

5. Vorrichtung nach Anspruch 4, **dadurch gekennzeichnet, dass** das mindestens eine Sensorsystem ein optisches Sensorsystem und/oder ein Beschleunigungssensor ist.

6. Vorrichtung nach einem der Ansprüche 4 oder 5, **dadurch gekennzeichnet, dass** das Sensorsystem Sensoren zur Lagebestimmung im dreidimensionalen Raum umfasst.

7. Vorrichtung nach Anspruch 4, **dadurch gekennzeichnet, dass** das Messgerät vertikal ausgerichtet und stabförmig ausgebildet ist.

8. Vorrichtung nach einem der Ansprüche 4 bis 7, **dadurch gekennzeichnet, dass** die Sensoren in ein flächiges Gebilde integriert sind und das flächige Gebilde nachfolgend lösbar auf einem stabförmigen Objekt anordbar ausgebildet ist

9. Verwendung eines computerimplementierten Verfahrens nach einem der Ansprüche 1 bis 3, zur Messung von Reaktionszeitverläufen sowie einer Vorrichtung nach einem der Ansprüche 4 bis 8 zur Durchführung eines Verfahrens nach einem der Ansprüche 1 bis 3 zur Messung von Reaktionszeitverläufen.

## Claims

1. Computer-implemented method for determining reaction time courses of at least one user by means of at least one sensor system, having the following steps:
i. presenting at least one virtual object in a virtual space,
ii. displaying a directed movement of the virtual object in a space,
wherein the movement proceeds along an axis which corresponds to the x-, y-, or z-axis of a Cartesian coordinate system, in relation to the user,
iii. determining or calculating the real temporal and spatial progression of the directed movement of the virtual object,
iv. detecting the user in real space,
v. detecting the movement or movement sequences of the user in real space, wherein at least one sensor system is designed for contactless detection of measurement data, wherein the at least one sensor system is an optical sensor system suitable for detection of movement sequences and at least one sensor system is suitable for gathering measurement data via the detection of contact by the user,
vi. determining or calculating the real temporal and spatial progression of the directed movement of the user,
vii. correlating the virtual and real movement sequences of steps iv and vi, and
viii. determining the reaction time courses using the movement sequences from steps iv and vi.

2. Computer-implemented method according to claim 1, **characterized in that** from the temporal and spatial progression of the directional movement of the virtual object and the temporal and spatial progression of the movement of at least one user, at least one starting point and at least one collision point of at least one virtual object and the user is determined.

3. Computer-implemented method according to claim 2, **characterized in that** the reaction time of the user is determined from the determined collision point.

4. Apparatus for determining reaction time courses of a real user in a virtual environment according to a method according to claims 1 through 3, comprising:
- at least one data processing unit,
- at least one sensor system,
- at least one display element suitable for presenting virtual objects in a virtual space,
- at least one output device suitable for outputting or relaying the determined sensory information,
- at least one interface to a database suitable for storing and for comparing the determined sensory data and at least one sensor system, wherein the sensor system for detecting the movement data is arranged in or on an additional real measuring device, wherein
- the sensor system is designed as a glove or is modelled, in terms of its shape, upon the virtual object in its presented shape.

5. Apparatus according to claim 4, **characterized in that** the at least one sensor system is an optical sensor system and/or an acceleration sensor.

6. Apparatus according to one of claims 4 or 5, **characterized in that** the sensor system comprises sensors for determining position in three-dimensional space.

7. Apparatus according to claim 4, **characterized in that** the measuring device is oriented vertically and rod-shaped.

8. Apparatus according to one of claims 4 through 7, **characterized in that** the sensors are integrated into a planar structure, and the planar structure is designed so as to subsequently be arrangeable detachably on a rod-shaped object.

9. Use of a computer-implemented method according to one of claims 1 through 3 for measuring reaction time courses, as well as use of an apparatus according to one of claims 4 through 8 for implementing a method according to one of claims 1 through 3 for measuring reaction time courses.

## Revendications

1. Procédé mis en œuvre par ordinateur pour la détermination des profils de temps de réaction d'au moins un utilisateur au moyen d'au moins un système de capteur, comprenant les étapes suivantes :
i. afficher l'au moins un objet virtuel dans un espace virtuel,
ii. afficher un mouvement dirigé de l'objet virtuel dans un espace, ce mouvement s'effectuant le long d'un axe correspondant à l'axe x, y ou z d'un système de coordonnées cartésiennes par rapport à l'utilisateur,
iii. déterminer ou calculer le profil temporel et spatial réel du mouvement dirigé de l'objet virtuel,
iv. détecter l'utilisateur dans l'espace réel,
v. détecter le mouvement ou les séquences de mouvements de l'utilisateur dans l'espace réel, au moins un système de capteur étant conçu pour la détection sans contact de données de mesure, l'au moins un système de capteur étant un système de capteur optiques adapté à la détection de séquences de mouvements, et au moins un système de capteur est adapté à la détection de données de mesure par détection du toucher de l'utilisateur,
vi. déterminer ou calculer le profil temporel et spatial réel du mouvement dirigé de l'utilisateur,
vii. corréler les séquences de mouvements virtuels et réels des étapes iv et vi et
viii. détermination des profils de temps de réaction à partir des séquences de mouvements des étapes iv et vi.

2. Procédé mis en œuvre par ordinateur selon la revendication 1, **caractérisé en ce qu'**à partir du profil temporel et spatial du mouvement dirigé de l'objet virtuel et du profil temporel et spatial du mouvement d'au moins un utilisateur au moins un point de départ et au moins un point de collision d'au moins un objet virtuel et l'utilisateur sont déterminés.

3. Procédé mis en œuvre par ordinateur selon la revendication 2, **caractérisé en ce que** le temps de réaction de l'utilisateur est déterminé à partir du point de collision déterminé.

4. Dispositif de détermination des profils de temps de réaction d'un utilisateur réel dans un environnement virtuel, selon un procédé selon les revendications 1 à 3, comprenant :
- au moins une unité de traitement de données,
- au moins un système de capteur,
- au moins un élément d'affichage adapté à l'affichage d'objets virtuels dans un espace virtuel,
- au moins un dispositif de sortie adapté à la sortie ou transmission des informations des capteurs,
- au moins une interface avec une base de données adaptée au stockage et à la comparaison des données sensorielles déterminées, et au moins un système de capteur, le système de capteur destiné à la détection des données de mouvement étant arrangé dans ou sur un dispositif de mesure réel additionnel,
- le système de capteurs étant conçu comme un gant ou le dispositif de mesure est modélisé sur l'objet virtuel tel qu'il est affiché.

5. Dispositif selon la revendication 4, **caractérisé en ce que** l'au moins un système de capteur est un système de capteur optiques et/ou un accéléromètre.

6. Dispositif selon la revendication 4 ou 5, **caractérisé en ce que** le système de capteur comprend des capteurs pour la détermination de position dans l'espace tridimensionnel.

7. Dispositif selon la revendication 4, **caractérisé en ce que** le dispositif de mesure est orienté verticalement et conçu en forme de bâton.

8. Dispositif selon l'une quelconque des revendications 4 à 7, **caractérisé en ce que** les capteurs sont intégrés dans une structure plane et que cette structure plane est ensuite conçu de manière amovible arrangeable sur un objet de forme de bâton.

9. Utilisation d'un procédé mis en œuvre par ordinateur selon l'une quelconque des revendications 1 à 3 pour la mesure des profils de temps de réaction, et d'un dispositif selon l'une quelconque des revendications 4 à 8 pour la mise en œuvre d'un procédé selon l'une quelconque des revendications 1 à 3 pour la mesure des profils de temps de réaction.
